(19) **Europäisches Patentamt**

**European Patent Office**

**Office européen des brevets**

(11) **EP 0 947 189 B1**

(12) **EUROPEAN PATENT SPECIFICATION**

(45) Date of publication and mention
of the grant of the patent:
**13.04.2005 Bulletin 2005/15**

(51) Int Cl.⁷: **A61K 7/00**, A61K 7/48,
C09J 139/06, C09J 171/02,
C09J 135/08

(21) Application number: **97934707.7**

(22) Date of filing: **06.08.1997**

(86) International application number:
**PCT/JP1997/002733**

(87) International publication number:
**WO 1998/006375 (19.02.1998 Gazette 1998/07)**

(54) **WET TACKY COMPOSITIONS, PLASTER PATCHES COMPRISING THE SAME, AND METHODS FOR USING THE SAME**

NASSE UND KLEBRIGE ZUSAMMENSETZUNGEN DIESELBEN ENTHALTENDE PFLASTER UND VERFAHREN ZUR ANWENDUNG DERSELBEN

COMPOSITIONS COLLANTES ET HUMIDE, TIMBRES A EMPLATRE COMPRENANT CELLES-CI ET PROCEDES D'UTILISATION ASSOCIES

(84) Designated Contracting States:
**AT BE CH DE DK ES FI FR GB GR IE IT LI LU MC NL PT SE**

(30) Priority: **13.08.1996 JP 21383596**
 **13.08.1996 JP 21384496**
 **13.08.1996 JP 21384896**
 **14.04.1997 JP 11340697**
 **14.04.1997 JP 11340797**
 **21.05.1997 JP 14872497**
 **30.06.1997 JP 620897 U**

(43) Date of publication of application:
**06.10.1999 Bulletin 1999/40**

(73) Proprietor: **NITTO DENKO CORPORATION**
**Osaka 567 (JP)**

(72) Inventors:
 • **SUGII, Tetsuji, Nitto Denko Corporation**
 **Ibaraki-shi Osaka 567 (JP)**
 • **YAMAMOTO, Katsuhiro,**
 **Nitto Denko Corporation**
 **Ibaraki-shi Osaka 567 (JP)**
 • **KONNO, Masayuki, Nitto Denko Corporation**
 **Ibaraki-shi Osaka 567 (JP)**
 • **KAWASAKI, Takashi**
 **Ibaraki-shi Osaka 567 (JP)**

(74) Representative: **Grünecker, Kinkeldey,**
**Stockmair & Schwanhäusser Anwaltssozietät**
**Maximilianstrasse 58**
**80538 München (DE)**

(56) References cited:
| JP-A- 1 038 007 | JP-A- 3 037 021 |
| JP-A- 4 054 107 | JP-A- 4 054 108 |
| JP-A- 8 092 054 | JP-A- 8 319 213 |
| JP-A- 61 254 512 | JP-U- 6 004 032 |

 • PATENT ABSTRACTS OF JAPAN vol. 012, no. 410 (C-540), 28 October 1988 (1988-10-28) & JP 63 146972 A (SEKISUI CHEM CO LTD), 18 June 1988 (1988-06-18)

## Description

Technical Field

The present invention relates to a composition which exhibits pressure-sensitive adhesion when wetted (hereinafter referred to "wet pressure-sensitive adhesive composition"), an adhesive sheet using the same and a method of using the adhesive sheet. More specifically, the present invention pertains to a wet pressure-sensitive adhesive composition which exhibits pressure-sensitive adhesion when it is moistened or wetted with water or a hydrophilic medium such as methyl alcohol or ethyl alcohol, an adhesive sheet using the wet pressure-sensitive adhesive composition and a method of using the adhesive sheet.

Background Art

For a skin covering material (medical material, such as dressing, to be applied to the skin) used in hospitals or the like, medical pressure-sensitive adhesives made of a natural rubber or acrylic ester have conventionally been employed to attain good adhesion to the skin. Such a medical pressure-sensitive adhesive is designed to exhibit good adhesion to the skin so that adhesion to a wet surface cannot be expected. For heightening the moisture permeability of the pressure-sensitive adhesive, a water-absorptive high molecule is added thereto or fine through-type pores are formed in the base or pressure-sensitive adhesive layer, which, however, cannot secure sufficient adhesion to the wet surface.

Various facial packs are also applied to the skin surface upon use. As a peel-off type facial pack, mainly used is that used by applying a film-forming agent to the skin surface, allowing it to stand for a predetermined time, thereby drying to form the film sufficiently and then peeling the film from the skin. As the product form, jelly, paste and powder are known. Such a facial pack is however accompanied with the drawback that it takes time to dry the film, that is, to leave the film to stand until the film formation, after the facial pack is applied to the skin.

A facial pack sheet in which a film forming agent has been formed into a sheet in advance is also known. It has two types, one is applied to an application site after the sheet is moistened or wetted by bringing it into contact with water or a hydrophilic medium and the other is applied after the application site is wetted with water or a hydrophilic medium in advance.

This type of a facial pack generally adopts a wet pressure-sensitive adhesive composition mainly composed of a water-soluble organic high molecule. This wet pressure-sensitive adhesive composition exhibits pressure-sensitive adhesion when moistened or wetted with water or a hydrophilic medium such as methyl alcohol or ethyl alcohol.

Here, when the water-soluble high molecule has an excessively large molecular weight, long hours are required until adhesion is exhibited through water or a hydrophilic medium. Although a water-soluble organic high molecule having a low molecular weight is, on the other hand, advantageous in the exhibition of adhesion within a short time through water or a hydrophilic medium, it is accompanied with the problems that since the composition has a low molecular weight and therefore has low mechanical strength under dry conditions, not only the dried film cannot be peeled off easily but also a peeling residue appears at the time of peeling.

In addition, the amount of water or a hydrophilic medium on the surface varies depending on the application site or time until the application. In order to attain stable application effects under any conditions, the thickness of the film formed from the above-described composition is generally adjusted to 100 to 300 $\mu$m, resulting in the problems that it takes time to dry the film after application and the film foams during drying, thereby deteriorating the film strength.

JP-A-63146972 relates to a hydrophilic tacky adhesive composition containing polyvinyl pyrrolidone and/or vinyl pyrrolidone-vinyl acetate copolymer; another polymer insoluble in water, but soluble in alcohol, preferably ethyl cellulose; and a softener, preferably glycerol, diglycerol, sorbitol or sorbitol derivatives.

JP-A-4054107 and JP-A-4054108 disclose a sheetlike packed agent with good and table adhesion, said agent having a backing supporting layer comprising a plastic film having softness, and a tacky plaster layer provided on the other face. The tacky plaster layer comprises a first component containing polyvinyl pyrrolidone and/or a copolymer containing vinyl pyrrolidone, and a second component comprising a low molecular weight component.

The present invention has been made in consideration of the above-described drawbacks of the prior art. An object of the present invention is therefore to provide a wet pressure-sensitive adhesive composition which exhibits pressure-sensitive adhesion through water or a hydrophilic medium, adheres suitably to the application site, dries speedily and has high mechanical strength after drying, can be peeled off easily and is suitable for the removal of horny plugs from the skin; an adhesive material formed of the composition; and a method of using the adhesive material.

Disclosure of the Invention

With a view to overcoming the above-described problems, the present inventors have carried out various investigations. As a result, it has been found that the above-described problems can be overcome unexpectedly by adding an inorganic filler to a specific water-soluble organic high molecule, leading to the completion of the present invention.

In the present invention, there is thus provided a wet pressure-sensitive adhesive composition comprising,

- as a component A, at least one compound of polyvinyl pyrrolidone and a vinyl pyrrolidone·vinyl acetate copolymer which is, at the same time, a mixture of a high-molecular weight polymer having a molecular weight of 1,000,000 to 5,000,000, and a low-molecular weight polymer having a molecular weight of 5,000 to 300,000;
- as a component B, 1 to 75 parts by weight per 100 parts by weight of component A, a liquid plasticizer compatible with component A; and
- as a component C, 10 to 200 parts by weight per 100 parts by weight of component A, an inorganic filler.

In another embodiment of the present invention, there is also provided an adhesive material comprising the above-described wet pressure-sensitive composition.

In a further embodiment of the present invention, there is also provided a nasal adhesive sheet comprising at least an adhesive layer containing the above-described wet pressure-sensitive composition.

In a still further embodiment of the present invention, there is also provided a method of using the above-described adhesive material.

Brief Description of the Drawings

FIGS. 1 to 7 are plan views each illustrating one mode of a nasal adhesive sheet according to the first embodiment of the present invention.

FIG. 8 is a plan view illustrating one mode of a nasal adhesive sheet according to the second embodiment of the present invention.

FIG. 9 is a view illustrating another mode of the above-described nasal adhesive sheet, wherein FIG. 9(a) is its plan view and FIG. 9(b) is its cross-sectional view.

FIG. 10 is a cross-sectional view of a further mode of the above-described nasal adhesive sheet.

FIG. 11 is a plan view illustrating a conventional nasal adhesive sheet.

In these figures, symbols represent following meanings respectively.

A: First nasal adhesive sheet according to the present invention
B: Second nasal adhesive sheet according to the present invention
11: Supporting layer
12: Adhesive layer
21: Slit (mark)
22, 22a, 22b: Wedge-shaped concave portions (mark)
23, 23a, 23b: Substantially semi-circular concave portions (mark)
24: Substantially semi-circular convex portion(mark)
31a, 31b: Prints (mark)
32: Protrusion (mark)
33: Recess (mark)

Best Modes for Carrying Out the Invention

In the present invention, when an adhesive material is produced using a wet pressure-sensitive adhesive composition having the component C incorporated therein, the adhesive layer has improved mechanical strength at the time when it is moistened or wetted and made tacky by water or a hydrophilic medium and then dried. This makes it possible to select, as the component A, a low-molecular weight material which is easily moistened and exhibits tackiness readily from wider range of materials. In addition, time required for drying after application can be shortened by the incorporation of the component C so that even if the adhesive layer is thick, it does not take so long hours for drying as usual. The reason why the adhesive material of the present invention can be dried in a shorter time compared with the conventional one is presumed that the interface between the component C and both of the components A and B serves as a passage for the escape of water.

Among the components of the wet pressure-sensitive adhesive composition of the present invention, the component A imparts tackiness to the composition by the existence of water or a hydrophilic medium and as the component A, at least one compound selected from polyvinyl pyrrolidone and a vinyl pyrrolidone·vinyl acetate copolymer, are used.

The component A is a mixture of a high molecular weight polymer and a low molecular weight polymer as set forth above. The term "molecular weight" as used herein means a weight average molecular weight. If a high molecular weight polymer is used, the adhesive layer available from the wet pressure-sensitive adhesive composition having the above-described composition has a merit in that it has strong mechanical strength after applied and dried and the amount of the adhesive residue (remaining amount after peeling) is small. When it is wetted with water, however, it takes time for the polymer, as a facial pack sheet, on the surface of the adhesive layer to dissolve or it does not dissolve but rather swells and sometimes does not reach the horny plug existing deeply in the pore of the skin sufficiently. When a low molecular weight polymer is used, on the other hand, it is effective in speedy dissolution of the polymer on the surface of the adhesive layer and easy arrival even at the part of the horny plug. It however happens to take time to sufficiently dry the surface of the adhesive layer which has contained water or hydrophilic medium employed. Even if the surface is dried sufficiently, water remains in the adhesive layer and deteriorates the mechanical strength. The adhesive tends to remain upon peeling, sometimes resulting in the unsatisfactory removal of the horny plug. In order to evaporate water sufficiently to prevent the remaining of the adhesive, the adhesive material must be applied continuously for long hours. It is considerably painful to stay for a long time with the adhesive material being applied until the adhesive material becomes sufficiently dry. Generally, it is possible to endure waiting for 5 to 30 minutes, but not for more than 30 minutes.

Thus, polyvinyl pyrrolidone or a vinyl pyrrolidone·vinyl acetate copolymer are used as the component A, by using a mixture of a high molecular weight polymer having an average molecular weight of 1,000,000 to 5,000,000 and a low molecular weight polymer having an average molecular weight of 5,000 to 300,000, in order to provide a wet pressure-sensitive adhesive composition making the best use of the features of the both polymers. In this case, from the viewpoint of heightening the mechanical strength after drying, it is desired to incorporate a high molecular weight vinyl pyrrolidone·vinyl acetate copolymer in a low molecular weight polyvinyl pyrrolidone or in a mixture of a high molecular polyvinyl pyrrolidone and a low molecular weight polyvinyl pyrrolidone.

Although the mixing ratio of the high molecular weight polymer to the low molecular weight polymer varies depending on the molecular weight of the polymers employed or the use purpose, the ratio by weight of the high molecular weight polymer to the low molecular weight polymer is from 5:95 to 95:5, preferably 20:80 to 70:30. It is more preferred to make the proportion of the low molecular weight polymer larger from the viewpoint of removal efficiency of horny plugs and reduction of a pain upon peeling.

As the component B to be used in the present invention, a material which has compatibility with the component A and dissolves it, thereby exhibiting plasticizing effects is employed. Examples include polyethylene glycols such as ethylene glycol, diethylene glycol, triethylene glycol and hexamethylene glycol; polypropylene glycols such as polypropylene glycol and dipropylene glycol; polyglycerins such as glycerin and diglycerin; butylene glycols such as 1,3-butylene glycol and 1,4-butylene glycol; sugar alcohols such as sorbitol and mannitol; and glycerides such as lanolin, lecithin and olive oil. They can be used either singly or in combination as the component B.

As the component C, various inorganic fillers which are sparingly soluble or insoluble in a mixture of the components A and B are employed. Examples of the inorganic filler include inorganic or complex oxides such as silica, alumina, zinc oxide, titanium oxide, talc, clay, kaolin and glass; inorganic compounds such as barium sulfate, calcium carbonate, hydroxy apatite, ceramics and carbon; and metals and alloys of various metals. The above-exemplified filler is usually preferred to be used in the powdery form. Although no particular limitation is imposed on the appearance, globular powder is preferred from the viewpoint of the uniform dispersion. The powder preferably has an average particle size of 0.01 to 50 $\mu$m, particularly 0.1 to 10 $\mu$m. They can be used either singly or in combination as the component C.

If the use amount of the component B to the component A is too large, after the adhesive layer obtained from the wet pressure-sensitive adhesive composition of the present invention is applied and dried, the mechanical strength becomes insufficient. On the other hand, if it is too small, the flexibility of the adhesive layer obtained from the wet pressure-sensitive adhesive composition of the present invention becomes poor, which makes it difficult to well-fit the adhesive layer to the application site. Accordingly, the amount of the component B is 1 to 75 parts by weight, more preferably 5 to 50 parts by weight per 100 parts by weight of the component A.

When the use amount of the component C to the component A is too large, the adhesive layer available from the wet pressure-sensitive adhesive composition of the present invention has poor flexibility, which makes it difficult to well-fit the adhesive layer to the application site. When the weight ratio is too small, on the contrary, the improving effects of the mechanical strength after the adhesive layer available from the wet pressure-sensitive adhesive composition of the present invention is applied and dried are poor and it takes more time to carry out drying after application. Accordingly, the component C is used in an amount of 10 to 200 parts by weight, preferably 25 to 100 parts by weight per 100 parts by weight of the component A.

The adhesive material according to the present invention comprises at least an adhesive layer containing the wet pressure-sensitive adhesive composition of the present invention. The adhesive material can be produced as a sheet composed solely of the adhesive layer, but it is preferred to form a supporting layer on one side of the adhesive material from the viewpoints that not only such a constitution generally facilitates the handling of it but also peeling property from the application site shows a further improvement owing to the improvement in the mechanical strength of the adhesive material.

There is no particular limitation imposed on the supporting layer insofar as it does not suppress the drying rate of the adhesive layer after application and flexibility before and after the application and contributes to an improvement in the mechanical strength. Various structures and various materials can be adopted. Examples of the structure include aggregates of fibers such as woven fabric, nonwoven fabric, knit fabric and paper; and sheets, for example, films such as porous film and air permeable film. Among these structures, those having stretchability sufficient for fitting to the curve of the application site are particularly preferred. Examples of the material include synthetic or natural organic high molecules such as nylon, polyester, polyethylene, polypropylene, polyurethane and cellulose.

The air permeability of the supporting layer to be used in the present invention is preferably 10 seconds or less as measured in accordance with the method of JIS P-8117. Insufficient air permeability of the supporting layer retards the drying of the applied adhesive layer, thereby presumably forming a peeling residue on the application site upon peeling. When the supporting layer having an air permeability not less than 10 seconds is used, it takes time for drying and at the same time a peeling residue appears, which presumably makes it unsuitable for practical use. If the above-described preferable air permeability is secured, no particular limitation is imposed on the thickness of the supporting layer. In consideration of the flexibility of the adhesive layer or the like, however, its thickness is preferably 5 to 500 μm, with 10 to 200 μm being particularly preferred.

The supporting layer may exist in any one of the following forms: wherein the adhesive layer has been simply stacked on one side of the supporting layer, wherein the inside of the supporting layer is immersed with a part of the composition of the adhesive layer from the one side of the supporting layer; and wherein a part of the adhesive layer has oozed out from one side of the supporting layer.

The adhesive layer contains the above-described wet pressure-sensitive adhesive composition and is produced by applying a coating liquid composed of the wet pressure-sensitive adhesive composition and water and/or a hydrophilic medium such as ethyl alcohol or methyl alcohol to a sheet which will be the above-described supporting layer or a sheet which will be a releasing layer when the adhesive material has the releasing layer, followed by drying.

The adhesive material of the present invention exhibits tackiness by absorbing water or a hydrophilic medium applied to an application site or by absorbing water given in advance to the adhesive layer so that it is preferred to determine the content of water or hydrophilic medium in advance. The content ratio of water or hydrophilic medium in the adhesive layer, that is, the ratio of water or hydrophilic medium to the adhesive layer containing water or hydrophilic medium is preferably 25 wt.% or less, with 7 to 20 wt.% being particularly preferred. When the content exceeds 25 wt. %, absorption of water or hydrophilic medium to be added upon use tends to become inferior and sufficient adhesion effects are not always exhibited. In addition, the adhesive material itself becomes sticky to hands, thereby deteriorating handleability. When the content ratio of water or hydrophilic medium becomes 7 wt.% or less, it sometimes becomes impossible to fit it to the application site.

Although the adhesive layer is able to have improved mechanical strength and peeling property from the application site by using as the component A a mixture of two polymers different in the molecular weight, the mechanical strength under dry conditions after the application becomes too low or too high depending on the kinds or physical properties of the component A, B or C. It is therefore preferred to determine the mixing ratio as needed within the above-described amounts in consideration of the kinds or physical properties of them.

The amount of water or the hydrophilic medium to be added in advance to the adhesive layer or that to be applied in advance to the application site varies depending on the use time. Accordingly, it is preferred that the thickness of the adhesive layer is adjusted to absorb water or the hydrophilic medium as much as possible. More specifically, it is preferred that the thickness is from 10 to 500 μm, particularly from 50 to 250 μm.

In the adhesive material of the present invention, it is preferred to stack a releasing layer on the adhesive layer, judging from that the adhesive layer can be stored under sanitary conditions or the adhesive material can be stored in piles or as a roll. No particular limitation is imposed on the releasing layer insofar as it can be used widely as a releasing layer for general adhesive materials. Examples thereof include various films and sheets, for example, plastic films such as polyester, polyethylene and polypropylene and metal films such as aluminum foil. It is also possible to use a woodfree paper, glassine paper, parchment paper or woodfree paper obtained by laminating polyethylene or the like on such a releasing paper.

It is preferred to dispose a releasing layer which has been treated, at its contact surface with the adhesive layer, with a releasing agent, because such a releasing layer can be adhered or peeled easily in repetition. There is no particular limitation imposed on the releasing agent insofar as it is a releasing agent generally employed. For example, silicone materials, which include dimethyl polysiloxane as a typical example, can be used. As the silicone releasing agent, either one of solvent type silicone or emulsion type silicone can be used. There are addition type and condensation type as a reaction type, with the addition type being generally employed.

By using such a releasing layer treated with a releasing agent, adhesion with the adhesive layer can be enhanced while adjusting the water content or the amount of the liquid component in the adhesive layer, which makes it possible to peel and apply the releasing layer in repetition. In addition, the adhesive material put on an improper place happens to stick firmly to the place under highly humid conditions, but this problem can be solved by the use of the releasing layer.

Moreover, a pain upon peeling the adhesive layer from the application site of a nose or the like can be relieved, which is presumed to be brought about by the partial transfer of the releasing agent to the surface of the adhesive layer and exhibition of easily-peeling effects by this transferred releasing agent. From the viewpoint of the transfer to the adhesive layer, polysiloxane having a lower molecular weight is preferred, with the emulsion type silicone being particularly preferred.

The adhesive material of the present invention having a supporting layer and a releasing layer can be produced, for example, according to the following process. The adhesive material can be obtained by adding the components B and C to the component A, stirring the resulting mixture uniformly, forming the reaction mixture into a liquid by adding water or a hydrophilic medium to give an amount of 20 to 70 wt.% for the adjustment of viscosity or concentration, applying the resulting liquid to the releasing layer so as to adjust the dry thickness within a desired range, drying as needed to form an adhesive layer, stacking thereon the supporting layer, and then drying the resulting laminate. Here, the drying is preferably carried out under the conditions set to adjust the content of water or the hydrophilic medium of the adhesive layer to 25 wt.% or less.

To the above-described liquid, cosmetics, perfumes, antiseptics, coloring agents, alcohols and ultraviolet absorbers, the other agents or additives ordinarily employed for adhesive materials can be added in an ordinary amount.

It is preferred to incorporate a keratolytic component as an additive, because an adhesive sheet suitable for the removal of a horny plug from the skin can be obtained by the incorporation. There is no particular limitation imposed on the keratolytic component insofar as it dissolves or softens the horny layer on the human skin surface. Specific examples include organic acids such as salicylic acid, citric acid, malic acid, lactic acid, tartaric acid, fumaric acid and succinic acid and esters thereof. They can be used either singly or in combination. As the esters of an organic acid, methyl ester, ethyl ester, and linear or branched butyl esters of the above-exemplified organic acid may be used mainly. Components easily soluble in water or a hydrophilic medium such as ethyl alcohol are desired. Most of the above-exemplified various organic acids are easily soluble in water or a hydrophilic medium and a solution of them in water or the hydrophilic medium applied to the surface of the adhesive layer or application site effectively dissolves or softens a horny layer. Besides such organic acids or esters thereof, various keratolytic components such as resorcin or thyroxolone can also be used.

Although depending on the kinds or properties of the keratolytic component or wet pressure-sensitive adhesive composition, the keratolytic component is added in an amount of 0.1 to 20 parts by weight, preferably 1 to 10 parts by weight per 100 parts by weight of the wet pressure-sensitive adhesive composition. When the amount is less than 0.1 part by weight, the horny layer cannot be dissolved or softened sufficiently and the keratolytic component cannot exhibit its effects fully. When the amount exceeds 20 wt.%, on the other hand, skin irritation due to the keratolytic component increases, which deteriorates feeling upon use. The use of an organic acid in such an excessive amount reduces the pH of the wet pressure-sensitive adhesive composition, resulting in a possibility of causing an adverse influence that the adhesive layer can not have sufficient tackiness.

The adhesive material thus obtained is cut into desired shapes and the releasing layer is peeled off just before use. It is stored under appropriate humidity conditions by hermetical sealing or a like means in order to prevent a reduction in the content of water or a hydrophilic medium in the adhesive layer which otherwise occurs by drying during storage.

The adhesive material is applied to the application site by peeling the releasing layer from the adhesive material before application; adding water or a hydrophilic medium to the adhesive layer to moisten or wet it or applying water or the hydrophilic medium to the application site; and then applying the adhesive material to the application site.

Such an adhesive material is effectively used as a peel-off type facial pack, a horny plug remover for smoothly removing horny plugs formed in the skin pores, a skin covering material for the moist or wet skin surface or the like.

In recent years, care of a so-called T zone from the forehead to the nasal line has attracted attentions. Above all, a care of the nasal part has attracted attentions and a number of nasal adhesive sheets obtained by forming a cosmetic composition into a sheet in advance have been developed because they can be used with ease. The adhesive material according to the present invention can be used similarly as a nasal adhesive sheet. Most of the nasal adhesive sheets are cut and formed into a predetermined shape so as to fit for the application to the nasal part. For example, the nasal adhesive sheet C has, as illustrated in FIG. 11, an upper end and a lower end which are substantially parallel each other, with the lower end being designed longer than the upper end. The left and right ends extend upward almost perpendicularly to the lower end and forms a curve at the upper part so that the left and right lower ends might just fit the wings of the nose.

Even if the nasal adhesive sheet has been cut into such a shape as fit for the application to the nasal part in advance, it is difficult to make the center of the sheet coincide with the nasal line and the sheet is inevitably shifted to one side. The nasal adhesive sheet cannot therefore cover one of the wings of the nose sufficiently.

According to the embodiment of the present invention, the adhesive sheet can be attached properly to the nasal part without causing a shift to a right side or a left side. The nasal adhesive sheet according to the first embodiment of the present invention is characterized by that at least a pair of positioning marks is disposed symmetrically relative to the center line of the nasal adhesive sheet at the upper end and/or lower end of the adhesive material. For example, a slit, a concave portion or a convex portion can be used as the mark. The nasal adhesive sheet according to the second embodiment of the present invention is characterized by that at least a pair of positioning marks is disposed symmetrically relative to the center line of the adhesive sheet at the upper and/or lower end portion of the adhesive material. In this case, a print, recess or projection can be used as the mark. When a supporting layer is used, the mark is disposed on the supporting layer.

A description will next be made on the preferred embodiments of the nasal adhesive sheet according to the present invention more specifically with reference to accompanying drawings.

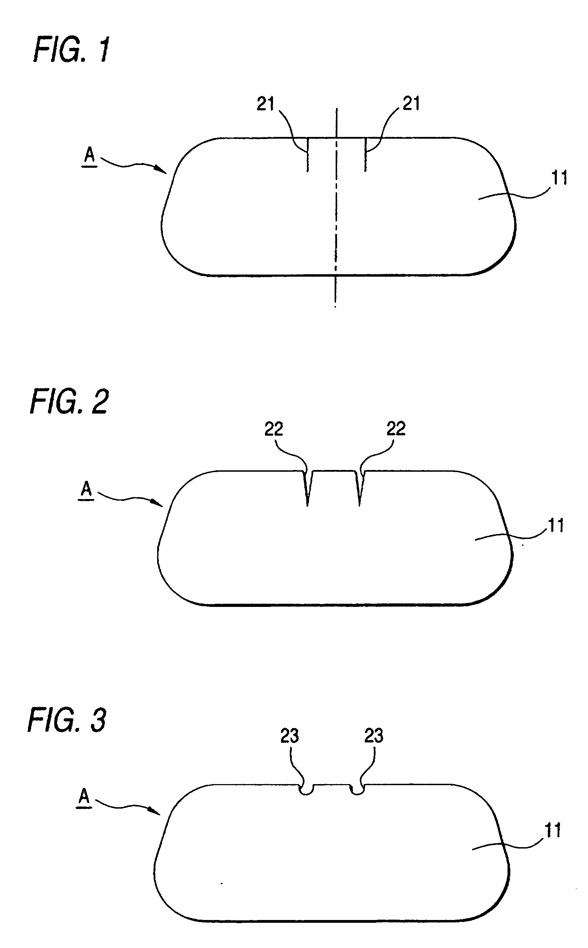

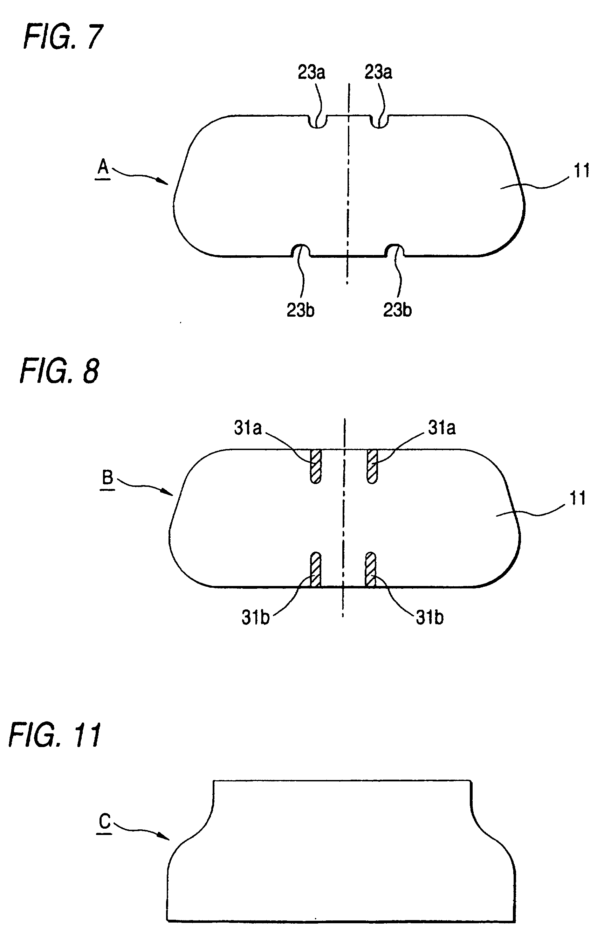

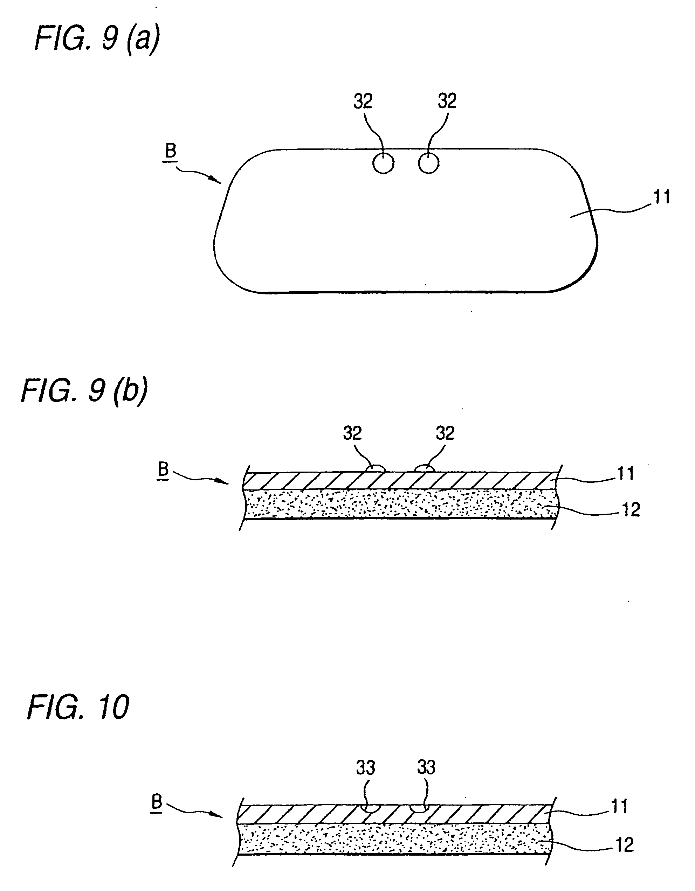

FIG. 1 is a plan view illustrating one mode of a nasal adhesive sheet A according to the first embodiment of the present invention; FIG. 2 is a plan view illustrating another mode of the nasal adhesive sheet A; FIGS. 3 to 7 are each a plan view illustrating a further mode of the nasal adhesive sheet A; FIG. 8 is a plan view illustrating one mode of a nasal adhesive sheet B according to the second embodiment of the present invention; FIGS. 9(a) and (b) illustrate another modes of the nasal adhesive sheet B, wherein (a) is its plan view and (b) is its cross-sectional view; and FIG. 10 is a cross-sectional view illustrating a further embodiment of the nasal adhesive sheet B.

The nasal adhesive sheet A of the first embodiment which is illustrated in FIGS. 1 to 7 has an adhesive layer 12, which is composed of the above-described wet pressure-sensitive adhesive composition, formed on the supporting layer 11 and as illustrated in FIGS. 1 to 7, it is cut and formed into a shape fit for the application to a nasal part.

As illustrated in each figure, the nasal adhesive sheet A has been cut almost linearly so that the upper end and the lower end are substantially parallel to each other and the lower end is formed longer than the upper end. The left and right side ends of the nasal adhesive sheet A are cut symmetrically to form a trapezoid. The upper side end from the upper end to the left or right side end and the lower side end from the left or right side end to the lower end are each rounded so that the sheet has roundish portions at both sides from the side end to the lower end. The nasal adhesive sheet A is cut into a size suited for the application to the nasal part and is designed so that the upper end exists at the upper part of the nasal line and each of the roundish portions from the side end to the lower end exists at one of the wings of the nose.

At the upper end and/or lower end of the nasal adhesive sheet A, marks for determining the proper application position are disposed. These marks are disposed symmetrically relative to the center line (indicated with a broken line in the figure) of the nasal adhesive sheet A.

Although the position of the marks differs slightly with the size of the face of the subject, it is disposed at almost a predetermined distance from the center line of the nasal adhesive sheet A irrespective of the size of the sheet A. Described specifically, it is suited to dispose left and right marks at the both sides of the nasal line when the upper end is positioned between the eyebrows above the nasal line.

Although there is no particular limitation imposed on the shape or size of the marks, the marks which do not reduce the application area or which can be felt with a fingertip are convenient. In particular, the marks which can be felt with a fingertip can be placed at both sides of the nasal line easily only by feeling the marks with a fingertip without confirming the application position in a mirror.

For example, the nasal adhesive sheet A as illustrated in FIG. 1 has a pair of left and right slits 21 at the upper end as marks. Although no particular limitation is imposed on the length of each slit 21, it is preferred to form so that it can be felt with the tips of two fingers, for example, a forefinger and middle finger.

The nasal adhesive sheet A is used in the following manner. After the nose or the surface of the adhesive layer 12 is wetted with water or a hydrophilic medium, the adhesive material is applied to the nose in such a way that the upper end is positioned between the eyebrows just above the nasal line. At this time, it is recommended to touch the slits 21 with two fingers and place the slits 21 at both sides of the nasal line. Then, the center of the lower end is positioned at the top of the nose while the central part of the nasal adhesive sheet A is pressed along the nasal line. The roundish portions at both sides of the sheet are pressed tightly against the wings of the nose, whereby the whole nasal adhesive sheet A is applied to the nose.

The nasal adhesive sheet A has, as described above, a pair of symmetrical marks (slits 21) so that the center of the sheet can be positioned at the nasal line and the adhesive sheet A can be applied properly to the nose without causing a shift to a left or right side. Marks in the form of slits 21 are accompanied with the advantages that they can be formed easily and do not reduce the application area.

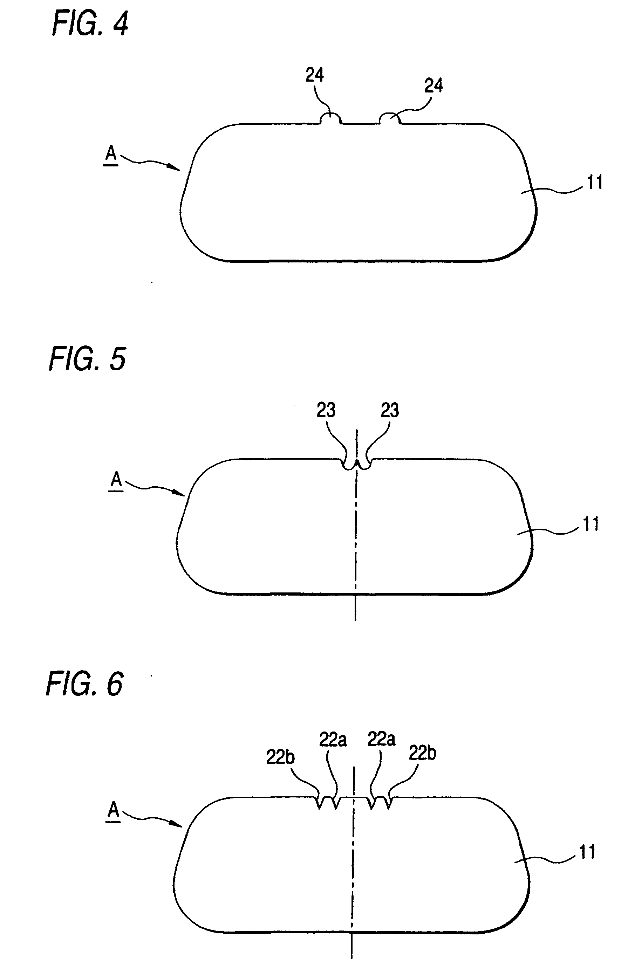

In the nasal adhesive sheet A as illustrated in FIG. 2, a wedge-shaped concave portion 22 is disposed at the upper end. The mark in the form of the wedge-shaped concave portion 22 can be felt more easily by a fingertip and the nasal adhesive sheet A can be attached well. Instead, it is possible to form the mark as a substantially semi-circular concave portion 23 as illustrated in the nasal adhesive sheet A of FIG. 3 or a substantially semi-circular convex portion 24 as illustrated in the nasal adhesive sheet A of FIG. 4. The marks in the form of the concave portions 22 and 23 or the convex portion 24 can be felt by a fingertip more easily compared with the slits 21. The convex portion 24 is particularly advantageous because it does not reduce the application area.

In the nasal adhesive sheet A as illustrated in FIG. 5, a pair of substantially semi-circular concave portions 23 are disposed symmetrically almost in contact with the center line. There is no particular limitation imposed on the position of the marks, and by disposing the marks symmetrically relative to the center line, the nasal adhesive sheet A can be positioned well from the both sides of the nasal line.

The marks are not limited to a pair of left and right ones, but plural pairs can be disposed. In the nasal adhesive sheet A as illustrated in FIG. 6, two pairs of the left and right wedge-shaped concave portions 22a, 22b are disposed at the upper end. The width of the nose depends on persons, and marks must be disposed in accordance with the size of the face of the subject. If plural pairs of marks (wedge-shaped concave portions 22a, 22b) are disposed in advance as the above-described nasal adhesive sheet A, the positions of the marks suited to the width of the nose can be felt with a fingertip, which makes it possible to attach the sheet A more suitably.

In the nasal adhesive sheet A as illustrated in FIG. 7, pairs of substantially semi-circular concave portions 23a and 23b are disposed at the upper end and lower end, respectively. The distance between the two concave portions 23b at the lower end is almost the same with or wider than that between the two concave portions 23a at the upper end and the concave portions 23b at the lower end are disposed so as to exist at both sides of the nose top upon adhesion. Since a pair of marks (concave portions 23b) is also disposed symmetrically relative to the center line at the lower end, it is possible to position the lower end suitably, which facilitates the positioning of the nasal sheet adhesive material. Needless to say, disposal of plural pairs of marks at the lower end, which is not illustrated though, is more preferred. An object of the present invention is achieved sufficiently even by the disposal of marks only at the lower end.

The nasal adhesive sheet B according to the second embodiment which is illustrated in FIGS. 8 to 10 is composed of a supporting layer 11 and an adhesive layer 12 similar to the nasal adhesive sheet according to the first embodiment. It is cut and formed into a shape suited for the application to a nose. This nasal adhesive sheet according to the second invention differs only in that a pair of positioning marks is disposed symmetrically relative to the center line (indicated by a broken line in the diagram) of the nasal adhesive sheet B at the upper end portion and/or lower end portion thereof.

In the nasal adhesive sheet B as illustrated in FIG. 8, prints 31a and 31b are disposed as marks at the upper and lower end portions on the supporting layer 11. Although there is no particular limitation imposed on the size or shape of these prints 31a and 31b, the print 31a at the upper end portion is preferably disposed between eyebrows above the nasal line and the print 32b at the lower end portion is preferably disposed at both sides of the nose top. The prints 31a and 31b can also be disposed, as illustrated, to be in contact with the upper end and lower end, respectively or they can be disposed apart from the upper end or lower end as the protrusion 32 illustrated in FIG. 9. From the viewpoint of the easy application, however, it is preferred to dispose the prints to be in contact with the upper end. In this way, the prints 31a and 31b can be disposed on the supporting layer 11 as marks without decreasing the application area.

As illustrated in the nasal adhesive sheet B of FIG. 9, it is also possible to form a protrusion 32 as a mark on the surface of the supporting layer 11. The protrusion can be formed by applying spots of an ink on the sheet or by using an expandable ink. It can also be formed by sticking or adhering organic or inorganic particles or powders to the sheet. Examples of the organic ones include particles or powders formed of a natural or synthetic material, of which the synthetic material being preferred because particles or powders stable in quality are available therefrom. Examples of the synthetic material include particles or powders prepared from a thermoplastic resin or a thermosetting resin. Such particles or powders are stuck or adhered to the sheet by thermal fusion or by using a hot-melt resin.

As in the nasal adhesive sheet B illustrated in FIG. 10, a recess 33 may be formed on the supporting layer 11. The recess 33 can be formed, for example, by pressing the surface of the supporting layer 11 of the nasal adhesive sheet B cut and formed into a predetermined shape with a roll or press plate.

If the supporting layer 11 is not necessary, the mark can be disposed directly on the surface of the adhesive layer 12. When the mark is in the form of the recess 33 or protrusion 32, the nasal adhesive sheet B can be applied to the nose more easily compared with the mark in the form of print 31a or 31b. Another merit is that since the sheet can be marked in an easy manner, a cost increase can be suppressed.

The present invention will hereinafter be described by examples, comparative examples and referential examples, in which all designations of "%" and "part" or "parts" mean wt.% and part or parts by weight unless otherwise specified. The water content in the adhesive layer, thickness of the adhesive layer and air permeability of the supporting layer were measured as described below.

(Water Content in the Adhesive Layer)

From each of the adhesive materials produced in Examples and Comparative Examples, the adhesive layer of a predetermined size was peeled and the weight (Wo) of the adhesive layer so peeled was measured. Immediately after drying at 75°C for 30 minutes in a drier, the dry weight (Wt) was measured and the water content was determined from the following equation:

$$\text{Water content (\%)} = (Wo - Wt) / Wo \times 100$$

(Thickness of the Adhesive Layer)

It was measured by a dial gauge.

(Air Permeability of the Supporting Layer)

It was measured in accordance with JIS P-8117.

Reference Example 1

A material composed of 50% of polyvinyl pyrrolidone having an average molecular weight of 1,200,000, 15% of glycerin, 35% of titanium white and an appropriate quantity of water was stirred and mixed, whereby a liquid material was obtained. The resulting liquid material was applied uniformly onto a polyester film having a thickness of 50 μm and serving as a releasing layer, followed by stacking of a polyester nonwoven fabric having a basic weight of 25 g/m$^2$ and air permeability of 1.5 sec. After drying, an adhesive sheet having a three-layer structure was obtained. Incidentally, the thickness and water content of the adhesive layer were 200 μm and 25%, respectively

Reference Example 2

In the same manner and conditions as in Reference Example 1 except for the use of 58% of polyvinyl pyrrolidone having an average molecular weight of 220,000, 7% of sorbitol, 33% of Aerosil and 2% of titanium oxide, an adhesive sheet having a three-layer structure was obtained. Incidentally, the thickness and water content of the adhesive layer were 150 μm and 7.0%, respectively.

Reference Example 3

In the same manner and conditions as in Reference Example 1 except for the use of 60% of a vinyl pyrrolidone - vinyl acetate copolymer having an average molecular weight of 40,000, 5% of 1,4-butanediol, 33% of Aerosil and 2% of titanium oxide, an adhesive sheet having a three-layer structure was obtained. Incidentally, the thickness and water content of the adhesive layer were 150 μm and 8.0%, respectively.

Comparative Example 1

In the same manner and conditions as in Reference Example 1 except that glycerin was not used, an adhesive sheet having a three-layer structure was obtained. Incidentally, the thickness and water content of the adhesive layer were 200 μm and 10%, respectively.

Comparative Example 2

In the same manner and conditions as in Reference Example 1 except that titanium white was not added, an adhesive sheet having a three-layer structure was obtained. Incidentally, the thickness and water content of the adhesive layer were 200 μm and 7.5%, respectively.

Comparative Example 3

In the same manner and conditions as in Reference Example 1 except for using polyvinyl alcohol having an average polymerization degree of 500 instead of polyvinyl pyrrolidone, an adhesive sheet having a three-layer structure was obtained. Incidentally, the thickness and water content of the adhesive layer were 150 μm and 10%, respectively.

Reference Example 4

A material composed of 60% of polyethylene oxide having an average molecular weight of 750,000, 20% of glycerin, 20% of titanium white and an appropriate quantity of water was stirred and mixed, whereby a liquid material was obtained. The resulting liquid material was applied uniformly onto a polyester film having a thickness of 50 μm and serving as a releasing layer, followed by stacking of a polyester nonwoven fabric having a basic weight of 25 g/m² and air permeability of 1.5 sec. After drying, an adhesive sheet having a three layer structure was obtained. Incidentally, the thickness and water content of the adhesive layer were 150 μm and 5.0%, respectively.

Reference Example 5

In the same manner and conditions as in Reference Example 4 except for using 55% of polyethylene oxide having an average molecular weight of 200,000, 10% of sorbitol, 30% of Aerosil and 5% of titanium oxide, an adhesive sheet having a three-layer structure was obtained. Incidentally, the thickness and water content of the adhesive layer were 150 μm and 3.5%, respectively.

Reference Example 6

In the same manner and conditions as in Reference Example 4 except for using 57.5% of polyethylene oxide having an average molecular weight of 100,000, 7.5% of 1,4-butanediol, 30% of Aerosil and 5% of titanium oxide, an adhesive sheet having a three-layer structure was obtained. Incidentally, the thickness and water content of the adhesive layer were 150 μm and 4.0%, respectively.

Comparative Example 4

In the same manner and conditions as in Reference Example 4 except that glycerin was not used, an adhesive sheet having a three-layer structure was obtained. Incidentally, the thickness and water content of the adhesive layer were 150 μm and 3.5%, respectively.

Comparative Example 5

In the same manner and conditions as in Reference Example 4 except that titanium white was not added, an adhesive sheet having a three-layer structure was obtained. Incidentally, the thickness and water content of the adhesive layer were 150 μm and 5%, respectively.

Comparative Example 6

In the same manner and conditions as in Reference Example 4 except for using polyvinyl alcohol having an average polymerization degree of 500 instead of polyethylene oxide, an adhesive sheet having a three-layer structure was obtained. Incidentally, the thickness and water content of the adhesive layer were 150 μm and 10%, respectively.

Reference Example 7

A material composed of 62% of the butyl ester of a methyl vinyl ether·maleic anhydride copolymer having an average molecular weight of 20,000, 3% of glycerin, 35% of titanium white and an appropriate quantity of water was stirred and mixed, whereby a liquid material was obtained. The resulting liquid material was applied uniformly onto a polyester film having a thickness of 50 μm and serving as a releasing layer, followed by stacking a polyester nonwoven fabric having a basic weight of 25 g/m² and air permeability of 1.5 sec. After drying, an adhesive sheet having a three-layer structure was obtained. Incidentally, the thickness and water content of the adhesive layer were 200 μm and 8%, respectively.

Reference Example 8

In the same manner and conditions as in Reference Example 7 except for the use of 60% the butyl ester of a methyl vinyl ether·maleic anhydride copolymer having an average molecular weight of 20,000, 5% of sorbitol, 33% of Aerosil and 2% of titanium oxide, an adhesive sheet having a three-layer structure was obtained. Incidentally, the thickness and water content of the adhesive layer were 200 μm and 5.5%, respectively.

Reference Example 9

In the same manner and conditions as in Reference Example 7 except for the use of 55% of the ethyl ester of a methyl vinyl ether·maleic anhydride copolymer having an average molecular weight of 20,000, 10% of 1,4-butanediol, 33% of Aerosil and 2% of titanium oxide, an adhesive sheet having a three-layer structure was obtained. Incidentally, the thickness and water content of the adhesive layer were 150 μm and 5.0%, respectively.

Comparative Example 7

In the same manner and conditions as in Reference Example 7 except that glycerin was not used, an adhesive sheet having a three-layer structure was obtained. Incidentally, the thickness and water content of the adhesive layer were 150 μm and 8%, respectively.

Comparative Example 8

In the same manner and conditions as in Reference Example 7 except that titanium white was not added, an adhesive sheet having a three-layer structure was obtained. Incidentally, the thickness and water content of the adhesive layer were 150 μm and 8.5%, respectively.

Comparative Example 9

In the same manner and conditions as in Reference Example 7 except for using polyvinyl alcohol having an average polymerization degree of 500 instead of the butyl ester of a methyl vinyl ether-maleic anhydride copolymer, an adhesive sheet having a three-layer structure was obtained. Incidentally, the thickness and water content of the adhesive layer were 150 μm and 10%, respectively.

The releasing strength and practical performance of each of the three-layer adhesive sheets which had been obtained in Reference Examples 1 to 9 and Comparative Examples 1 to 9 were evaluated. The results are shown in Table 1.

(Peeling Strength of Adhesive Material)

Onto a horizontally disposed bakelite plate, 0.5 ml of distilled water was poured dropwise and a sample of 2 cm x 5 cm square was bonded thereto quietly. The resulting laminate was left to stand for 30 minutes. Then, pulling was effected at a tensile rate of 300 mm/min. The peeling strength at the time was measured.

(Practical Performance of Adhesive Material)

After the nasal site of a volunteer to be evaluate practical performance was wetted with an appropriate amount of distilled water, a sample of 3 cm x 7 cm square was applied thereto. The sample was left to stand for 30 minutes and then peeled off. Through this test, adhesion, dryness, strength of the film and residual amount after peeling were measured and they were evaluated by the following ratings.

◎ : excellent, ○: good, Δ: poor, x: very poor

## Table 1

| | | Peeling strength (Unit: g/cm² width) | Practical performance | | | |
|---|---|---|---|---|---|---|
| | | | Adhesion | Dryness | Film strength | Residual amount after peeling |
| Ref. | Example 1 | 500 | ◎ | ◎ | ◎ | ◎ |
| | Example 2 | 450 | ◎ | ◎ | ◎ | ◎ |
| | Example 3 | 350 | ◎ | ○ | ○ | ○ |
| | Comp. Ex. 1 | 700 | Δ | ○ | Δ | ○ |
| | Comp. Ex. 2 | 250 | Δ | x | Δ | x |
| | Comp. Ex. 3 | 150 | x | Δ | ○ | ○ |
| Ref. | Example 4 | 420 | ◎ | ◎ | ◎ | ◎ |
| | Example 5 | 350 | ◎ | ◎ | ○ | ○ |
| | Example 6 | 300 | ◎ | ○ | ○ | ○ |
| | Comp. Ex. 4 | 500 | Δ | ○ | Δ | ○ |
| | Comp. Ex. 5 | 200 | Δ | x | Δ | x |
| | Comp. Ex. 6 | 150 | x | Δ | ○ | ○ |
| Ref. | Example 7 | 430 | ◎ | ◎ | ◎ | ◎ |
| | Example 8 | 350 | ◎ | ○ | ○ | ○ |
| | Example 9 | 500 | ◎ | ○ | ○ | ○ |
| | Comp. Ex. 7 | 520 | Δ | ○ | Δ | ○ |
| | Comp. Ex. 8 | 300 | Δ | x | Δ | x |
| | Comp. Ex. 9 | 150 | x | Δ | ○ | ○ |

Example 10

| | |
|---|---|
| Polyvinyl pyrrolidone (weight average molecular weight: 160,000) | 6 parts |
| Polyvinyl pyrrolidone (weight average molecular weight: 1,200,000) | 4 parts |
| Water | 16 parts |
| Glycerin | 1.2 parts |
| Titanium oxide | 0.31 part |
| Silicon dioxide | 6 parts |

The above-described components were each weighed to a predetermined amount, followed by stirring and mixing, whereby a liquid material was obtained. The resulting liquid material was applied uniformly onto a polyester film having a thickness of 50 μm and serving as a releasing layer, followed by stacking a polyester nonwoven fabric having a basic weight of 25 g/m² and air permeability of 1.5 sec. After drying, a three-layer adhesive sheet having a supporting layer and a releasing layer was obtained. Incidentally, the thickness and water content of the adhesive layer were 150 μm and 15%, respectively.

Example 11

| | |
|---|---|
| Polyvinyl pyrrolidone (weight average molecular weight: 40,000) | 7 parts |
| Polyvinyl pyrrolidone (weight average molecular weight: 1,500,000) | 3 parts |
| Water | 16 parts |
| Glycerin | 1.2 parts |
| Titanium oxide | 0.31 part |

(continued)

| Silicon dioxide | 6 parts |
|---|---|

The above-described components were each weighed to a predetermined amount. As in Example 10, a three-layer adhesive sheet having a supporting layer and a releasing layer was produced. Incidentally, the thickness and water content of the adhesive layer were 150 μm and 15%, respectively.

Comparative Example 10

| Polyvinyl pyrrolidone (weight average molecular weight: 160,000) | 10 parts |
|---|---|
| Water | 16 parts |
| Glycerin | 1.2 parts |
| Titanium oxide | 0.31 part |
| Silicon dioxide | 6 parts |

The above-described components were each weighed to a predetermined amount. As in Example 10, a three-layer adhesive sheet having a supporting layer and a releasing layer was produced. Incidentally, the thickness and water content of the adhesive layer were 150 μm and 10%, respectively.

Comparative Example 11

| Polyvinyl pyrrolidone (weight average molecular weight: 120,000) | 10 parts |
|---|---|
| Water | 30 parts |
| Glycerin | 1.2 parts |
| Titanium oxide | 0.31 part |
| Silicon dioxide | 6 parts |

The above-described components were each weighed to a predetermined amount. As in Example 10, a three-layer adhesive sheet having a supporting layer and a releasing layer was produced. Incidentally, the thickness and water content of the adhesive layer were 150 μm and 15%, respectively.

The practical performance of each of the three-layer adhesive sheets obtained in Examples 10 and 11 and Comparative Examples 10 and 11 was evaluated in accordance with the below-described method and the results are shown in Table 2.

(Practical Performance of Adhesive material)

A panel of 20 females who had washed their faces or had not wore makeup were asked to apply the adhesive material of each of Examples to one of the two sides divided with the nasal line as a center and to apply the adhesive material of each of Referential Examples to the other side and feeling upon use of these adhesive materials were evaluated. Each adhesive material was cut into a piece of 3 cm x 4 cm and was applied after the nose site to be applied was wetted with an appropriate amount of water. After the adhesive material was left to stand as it was for 15 minutes, it was peeled from the nose. The removal degree of horny plugs, a residual amount after peeling and pain upon peeling were ranked in accordance with the below-described evaluation standards and an average score was determined. The above-described test was carried out on the adhesive materials of Example 10 and Comparative Example 10 first and then, on the adhesive materials of Example 11 and Comparative Example 11 some other day.

[Evaluation Standards]

(Removal Degree of Horny Plugs)

- Plugs were removed very much: 5
- Plugs were removed much: 4
- It is hard to say whether plugs were removed much or were not removed much: 3
- Plugs were not removed sufficiently: 2
- Plugs were not removed at all: 1

(Residual Amount on the Nose After Peeling)

- The adhesive material did not remain at all: 5
- The adhesive material remained slightly: 4
- The one-third of the adhesive material remained: 3
- The one-third to half of the adhesive material remained: 2
- At least half of the adhesive material remained: 1

(Pain Upon Peeling)

- The adhesive material was peeled off without pain: 4
- The adhesive material was peeled off without problems but a slight pain: 3
- The adhesive material was peeled off with a little pain: 2
- The adhesive material was peeled off with a serious pain: 1

Table 2

|  | Removal degree of horny plugs | Residual amount on the nose after peeling | Pain upon peeling |
|---|---|---|---|
| Example 10 | 3.5 | 4.4 | 3.0 |
| Example 11 | 3.3 | 4.5 | 2.9 |
| Comp. Example 10 | 2.7 | 2.8 | 3.4 |
| Comp. Example 11 | 2.1 | 4.9 | 2.1 |

As shown in Table 2, concerning the removal degree of horny plugs, the score of each of the adhesive materials of the present invention obtained in Examples 10 and 11 was about one point higher than that of each of the adhesive materials obtained in Comparative Examples 10 and 11. Concerning the residual amount on the nose after peeling, the adhesive of the adhesive material of Example 11 remained a little more than that of Comparative Example 11, but the score of the adhesive material of Example 10 was 1.6 points higher than that of Comparative Example 10. Concerning pain upon peeling, both of the adhesive materials of Example 10 and Example 11 had less pain than the adhesive material of Comparative Example 11.

Reference Example 12

A liquid material was obtained by stirring and mixing 50 parts of polyvinyl pyrrolidone having a weight-average molecular weight of 160,000, 6 parts of glycerin, 0.31 part of titanium oxide, 30 parts by silicon dioxide and an appropriate quantity of water. The resulting liquid material was uniformly applied onto a polyethylene film of 50 μm thick having a surface (contact surface with an adhesive layer) treated with an emulsion type silicone agent, followed by stacking a polyester nonwoven fabric having a basic weight of 25 g/m$^2$ and air permeability of 1.5 sec in accordance with JIS P-8117. After drying, an adhesive sheet having a three-layer structure was obtained.

Reference Example 13

In the same manner as in Reference Example 12 except for the use of 58 parts of polyvinyl pyrrolidone having a weight-average molecular weight of 40,000, 15 parts of propylene glycol, 33 parts of talc and 2 parts of titanium oxide, an adhesive sheet was obtained.

Reference Example 14

In the same manner as in Reference Example 12 except for the use of 60 parts of a vinyl pyrrolidone·vinyl acetate copolymer (the vinyl pyrrolidone / vinyl acetate weight ratio = 6/4) having a weight-average molecular weight of 40,000, 15 parts of 1,4-butanediol, 2.5 parts of silicon dioxide and 16 parts of titanium oxide, an adhesive sheet was obtained.

Example 15

In the same manner as in Reference Example 12 except for the use of 6 parts of polyvinyl pyrrolidone having a weight-average molecular weight of 160,000, 4 parts of polyvinyl pyrrolidone having a weight-average molecular weight of 1,200,000, 16 parts of water, 1.2 parts of glycerin, 0.31 part of titanium oxide and 6 parts of silicon dioxide, an adhesive sheet was obtained.

Example 16

In the same manner as in Reference Example 12 except for the use of 7 parts of polyvinyl pyrrolidone having a weight-average molecular weight of 40,000, 3 parts of polyvinyl pyrrolidone having a weight-average molecular weight of 1,500,000, 16 parts of water, 1.2 parts of glycerin, 0.3 part of titanium oxide and 6 parts of silicon dioxide, an adhesive sheet was obtained.

Comparative Example 12

A liquid material was obtained by stirring and mixing 50 parts of polyvinyl pyrrolidone having a weight-average molecular weight of 160,000, 6 parts of glycerin, 0.31 part of titanium oxide, 30 parts of silicon dioxide and an appropriate quantity of water. The resulting liquid material was uniformly applied onto a polyethylene film of 50 μm thick having a surface (contact surface with an adhesive layer) not treated with an emulsion type silicone agent, followed by stacking of a polyester nonwoven fabric having a basic weight of 25 g/m$^2$ and air permeability of 1.5 sec in accordance with JIS P-8117. After drying, an adhesive sheet having a three-layer structure was obtained.

Comparative Example 13

In the same manner as in Comparative Example 12 except for the use of 58 parts of polyvinyl pyrrolidone having a weight-average molecular weight of 40,000, 15 parts of propylene glycol, 33 parts of talc and 2 parts of titanium oxide, an adhesive sheet was obtained.

Comparative Example 14

In the same manner as in Comparative Example 12 except for the use of 60 parts of a vinyl pyrrolidone·vinyl acetate copolymer (the vinyl pyrrolidone / vinyl acetate weight ratio = 6/4) having a weight-average molecular weight of 40,000, 15 parts of 1,4-butanediol, 2.5 parts of silicon dioxide and 16 parts of titanium oxide, an adhesive sheet was obtained.

Comparative Example 15

In the same manner as in Comparative Example 12 except for the use of 6 parts of polyvinyl pyrrolidone having a weight-average molecular weight of 160,000, 4 parts of polyvinyl pyrrolidone having a weight-average molecular weight of 1,200,000, 16 parts of water, 1.2 parts of glycerin, 0.31 part of titanium oxide and 6 parts of silicon dioxide, an adhesive sheet was obtained.

Comparative Example 16

In the same manner as in Comparative Example 12 except for the use of 7 parts of polyvinyl pyrrolidone having a weight-average molecular weight of 40,000, 3 parts of polyvinyl pyrrolidone having a weight-average molecular weight of 1,500,000, 16 parts of water, 1.2 parts of glycerin, 0.31 part of titanium oxide and 6 parts of silicon dioxide, an adhesive sheet was obtained.

The adhesive sheets of Examples and Reference Examples 12 to 16 and Comparative Examples 12 to 16 were produced as described above and the effect of the treatment of the releasing layer with a releasing agent was confirmed. The production steps were carried out under the drying conditions wherein the water content (calculated from the weight change after drying at 75°C for 30 minutes) of the adhesive layer after drying was adjusted to 15 to 22% in Examples and Reference Examples 12 to 16 and 7 to 15% in Comparative Examples 12 to 16. If the water content was adjusted to high in Comparative Examples, viscosity appears in the adhesive layer, which prevents the peeling of the releasing layer from the adhesive layer at the initial stage.

(Repeated Peeling Test)

As a result of the test, it has been found that the releasing layer could be peeled off and adhered in repetition in the case of the adhesive sheets of Examples and Reference Examples 12 to 16, while the releasing layer once peeled off could not be adhered again in the case of the adhesive sheets of Comparative Examples 3 to 7.

(Storage Test)

The peeling property was evaluated after storage at 25°C and 75% R.H. for 24 hours. With respect to the adhesive sheets obtained in Comparative Examples 12 to 16, some releasing layers were not peeled off easily. With respect to the adhesive sheets obtained in Examples and Reference Examples 12 to 16, all the releasing layers were peeled off easily.

(Application Test)

A panel of 20 experts who had washed their faces or had not wore makeup were asked to apply each of the adhesive materials obtained in Examples to one of the two sides divided with the nasal line as a center and to apply each of the adhesive materials obtained in Comparative Examples to the other side and feeling upon use of these adhesive materials were evaluated. Each adhesive material was cut into a piece of 3 cm x 4 cm and was applied after the nose site to be applied was wetted with an appropriate amount of water. After the adhesive material was left to stand as it was for 15 minutes, it was peeled from the nose. The pain upon peeling was ranked in accordance with the below-described evaluation standards and an average score of all the experts was determined. The above-described test was carried out on the adhesive materials of Reference Example 12 and Comparative Example 12 first and then, in the same manner, the tests on the adhesive materials of Reference Example 13 and Comparative Example 13, those of Reference Example 14 and Comparative Example 14, those of Example 15 and Comparative Example 15 and those of Example 16 and Comparative Example 16 were carried out later, respectively.

(Evaluation Standards)

· The adhesive material was peeled off without a pain: 4
· The adhesive material was peeled off without problems but a slight pain: 3
· The adhesive material was peeled off with a little pain: 2
· The adhesive material was peeled off with a serious pain: 1

As a result, the average evaluation score of all the adhesive sheets of Comparative Examples 12 to 16 was 2.1, while that of all the adhesive sheets of Examples and Reference Examples 12 to 16 was 2.5. It has therefore been confirmed that the adhesive material of the present invention can relieve the pain upon peeling.

Reference Example 17

A liquid material for application was obtained by stirring and mixing 50 parts of polyvinyl pyrrolidone having a weight-average molecular weight of 1,200,000, 15 parts of glycerin, 35 parts of silicic anhydride, 1 part of salicylic

EP 0 947 189 B1

acid and an appropriate quantity of water. The resulting liquid material was uniformly applied onto a polyethylene film of 50 µm thick having a surface (contact surface with an adhesive layer) treated with a releasing agent, followed by stacking a polyester nonwoven fabric having a basic weight of 40 g/m². After drying, an adhesive sheet having a three-layer structure was obtained. The resulting adhesive sheet had an adhesive layer having a thickness of 300 µm and a water content of 22% (calculated from the weight change after drying at 75°C for 30 minutes, which will be applied hereinafter).

Comparative Example 17

In the same manner as in Reference Example 17 except that salicylic acid was not added, an adhesive sheet was obtained. The adhesive sheet had an adhesive layer of 300 µm thick and water content of 22%.
A panel of 10 experts were asked to practically use the adhesive sheets of Reference Example 17 and Comparative Example 17 and plug removability, drying characteristics and skin irritation were evaluated as described below.

[Evaluation Test]

Each of the adhesive sheets obtained in Example and Comparative Example was cut into a proper size and applied to the nose of a panel of experts wetted with water. After the adhesive material was left to stand for 10 minutes and dried, it was peeled off. At that time, the panel of experts was asked to evaluate the horny plug removability, drying characteristics and skin irritation in accordance with the below-described standards. An average score of each of the adhesive sheets was calculated. Incidentally, the test was carried out in the order of Reference Example 17 and Comparative Example 17 at an interval of three days.

[Evaluation Standards]

(Horny Plug Removability)

·    Horny plugs were removed well: 3
·    Horny plugs were removed: 2
·    Horny plugs were not removed: 1

(Drying Characteristics)

·    The adhesive material was dried well: 3
·    The adhesive material was dried: 2
·    The adhesive material was not dried: 1

(Skin Irritation)

·    The nose became reddish: 3
·    The nose became a little reddish: 2
·    The nose did not become reddish: 1

The evaluation results are shown in Table 3. As can be seen from Table 3, it has been found that horny plugs were removed more easily by the adhesive sheet of Reference Example 17 compared with that of Comparative Example 17 to which no horny plug-dissolving component had been added. By the addition of the horny plug-dissolving component, neither drying characteristics were influenced largely nor skin irritation was caused. Although not shown in Table 3, it has been found that the adhesive material of Example did not become inferior to that of Comparative Example in the residue of the adhesive.

Table 3

|  | Horny plug removability | Drying characteristics | Skin irritation |
|---|---|---|---|
| Reference Example 17 | 2.8 | 2.7 | 1.1 |
| Comparative Example 17 | 2.1 | 2.7 | 1.2 |

Industrial Application Possibility

The wet pressure-sensitive adhesive composition according to the present invention dries quickly, has sufficient mechanical strength after drying and permits the formation of an adhesive layer having good peeling property. The wet pressure-sensitive adhesive composition is useful as a peel-off type facial pack or a skin covering material for a moist or wet skin surface and in particular, can provide an adhesive material for the removal of horny plugs which can remove horny plugs effectively and does not cause pain so much upon peeling.

**Claims**

1.   A wet pressure-sensitive adhesive composition comprising,

   - as a component A, at least one compound of polyvinyl pyrrolidone and a vinyl pyrrolidone·vinyl acetate copolymer which is, at the same time, a mixture of a high-molecular weight polymer having a molecular weight of 1,000,000 to 5,000,000, and a low-molecular weight polymer having a molecular weight of 5,000 to 300,000;
   - as a component B, 1 to 75 parts by weight per 100 parts by weight of component A, a liquid plasticizer compatible with component A; and
   - as a component C, 10 to 200 parts by weight per 100 parts by weight of component A, an inorganic filler.

2.   An adhesive sheet which comprises an adhesive layer composed of the wet pressure-sensitive adhesive composition according to claim 1.

3.   The adhesive sheet according to claim 2, wherein said adhesive layer contains a keratolytic component.

4.   The adhesive sheet according to claim 3, wherein said keratolytic component is an organic acid.

5.   The adhesive sheet according to claim 2, wherein said adhesive layer further comprises water or a hydrophilic medium in an amount of 25 wt.% or less.

6.   The adhesive sheet according to claim 2 comprising a supporting layer in the form of a sheet.

7.   The adhesive sheet according to claim 6, wherein said supporting layer has an air permeability of 10 seconds or less as measured in accordance with JIS P-8117.

8.   The adhesive sheet according to claim 6, wherein a releasing layer was disposed on said adhesive layer.

9.   The adhesive sheet according to claim 8, wherein a contact surface between said releasing layer and adhesive layer has been treated with a releasing agent.

10.  The adhesive sheet according to claim 9, wherein said peeling agent is a silicone material.

11.  The adhesive sheet according to claim 7 used for the application to a nasal part.

12.  A nasal adhesive sheet according to claim 11, wherein at least a pair of positioning marks is disposed at the upper end and/or lower end of said adhesive sheet symmetrically relative to the center line of the adhesive sheet.

13.  A nasal adhesive sheet according to claim 11, wherein at least a pair of positioning marks is disposed at the upper end portion and/or lower end portion of the adhesive sheet symmetrically relative to the center line of the adhesive sheet.

**14.** A method of using an adhesive sheet according to claim 2, comprising making the surface of the adhesive layer of the adhesive sheet at a moistened or wet state prior to application by bringing into contact with water or a hydrophilic medium.

**15.** A method of using an adhesive sheet according to claim 2, which comprises wetting an application site with water or a hydrophilic medium in advance and applying the surface of the adhesive layer of the adhesive sheet to the application site.

**Patentansprüche**

**1.** Im feuchten bzw. nassen Zustand selbstklebende (druckempfindliche) Klebstoffzusammensetzung, die umfasst

- als Komponente A mindestens eine Verbindung aus der Gruppe Polyvinylpyrrolidon und Vinylpyrrolidon/ Vinylacetat-Copolymer, die gleichzeitig ein Gemisch aus einem Polymer mit einem hohen Molekulargewicht, das ein Molekulargewicht von 1 000 000 bis 5 000 000 aufweist, und einem Polymer mit einem niedrigen Molekulargewicht, das ein Molekulargewicht von 5 000 bis 300 000 aufweist, darstellt;
- als Komponente B 1 bis 75 Gew.-Teile, bezogen auf 100 Gew.-Teile der Komponente A, eines flüssigen Weichmachers, der mit der Komponente A kompatibel ist, und
- als Komponente C 10 bis 200 Gew.-Teile, bezogen auf 100 Gew.-Teile der Komponente A, eines anorganischen Füllstoffs.

**2.** Klebefolie, die eine Klebstoffschicht umfasst, die besteht aus der im feuchten bzw. nassen Zustand selbstklebenden (druckempfindlichen) Klebstoffzusammensetzung nach Anspruch 1.

**3.** Klebefolie nach Anspruch 2, in der die Klebstoffschicht eine keratolytische Komponente enthält.

**4.** Klebefolie nach Anspruch 3, in der die keratolytische Komponente eine organische Säure ist.

**5.** Klebefolie nach Anspruch 2, in der die Klebstoffschicht außerdem Wasser oder ein hydrophiles Medium in einer Menge von 25 Gew.-% oder weniger umfasst.

**6.** Klebefolie nach Anspruch 2, die eine Trägerschicht in Form einer Folie umfasst.

**7.** Klebefolie nach Anspruch 6, in der die Trägerschicht eine Luftdurchlässigkeit von 10 s oder weniger, bestimmt gemäß JIS P-8117, aufweist.

**8.** Klebefolie nach Anspruch 6, in der eine Trennschicht auf der Klebstoffschicht angeordnet ist.

**9.** Klebefolie nach Anspruch 8, in der eine Kontaktoberfläche zwischen der Trennschicht und der Klebstoffschicht mit einem Trennmittel behandelt worden ist.

**10.** Klebefolie nach Anspruch 9, in der das Trennmittel (Abziehmittel) ein Silicon-Material ist.

**11.** Klebefolie nach Anspruch 7, die für das Aufbringen auf einen Nasenabschnitt verwendet wird.

**12.** Nasen-Klebefolie nach Anspruch 11, in der mindestens ein Paar von Positionsmarkierungen an dem oberen Ende und/oder dem unteren Ende der Klebefolie symmetrisch zu der Mittellinie der Klebefolie angeordnet ist.

**13.** Nasen-Klebefolie nach Anspruch 11, in der mindestens ein Paar von Positionsmarkierungen an dem oberen Endabschnitt und/oder dem unteren Endabschnitt der Klebefolie symmetrisch zu der Mittellinie der Klebefolie angeordnet ist.

**14.** Verfahren zur Verwendung einer Klebefolie nach Anspruch 2, das umfasst das Überführen der Oberfläche der Klebstoffschicht der Klebefolie in einen angefeuchteten oder nassen Zustand vor dem Aufbringen durch Inkontaktbringen derselben mit Wasser oder einem hydrophilen Medium.

**15.** Verfahren zur Verwendung einer Klebefolie nach Anspruch 2, das umfasst das vorherige Anfeuchten einer Auftragsstelle mit Wasser oder einem hydrophilen Medium und das Aufbringen der Oberfläche der Klebstoffschicht der Klebefolie auf die Auftragsstelle.

**Revendications**

1. Composition adhésive sensible à la pression humide comprenant,

   - en tant que composant A, au moins un composé de polyvinylpyrrolidone et un copolymère de vinylpyrrolidone-acétate de vinyle qui est, en même temps, un mélange d'un polymère de poids moléculaire élevée ayant une poids moléculaire de 1 000 000 à 5 000 000, et un polymère de poids moléculaire faible ayant une poids moléculaire de 5 000 à 300 000 ;
   - en tant que composant B, 1 à 75 parties en poids par 100 parties en poids d'un composant A, un plastifiant liquide compatible avec le composant A ; et
   - en tant que composant C, 10 à 200 parties en poids pour 100 parties en poids d'un composant A, une charge inorganique.

2. Feuille adhésive qui comprend une couche adhésive composée d'une composition adhésive sensible à la pression humide selon la revendication 1.

3. Feuille adhésive selon la revendication 2, dans laquelle ladite couche adhésive contient un composant kératolytique.

4. Feuille adhésive selon la revendication 3, dans laquelle ledit composant kératolytique est un acide organique.

5. Feuille adhésive selon la revendication 2, dans laquelle ladite couche adhésive comprend de plus de l'eau ou un milieu hydrophile en une quantité de 25 % en poids ou moins.

6. Feuille adhésive selon la revendication 2 comprenant une couche de support sous la forme d'une feuille.

7. Feuille adhésive selon la revendication 6, dans laquelle ladite couche de support a une perméabilité à l'air de 10 secondes ou moins comme mesuré conformément à JIS P-8117.

8. Feuille adhésive selon la revendication 6, dans laquelle une couche de libération a été disposée sur ladite couche adhésive.

9. Feuille adhésive selon la revendication 8, dans laquelle une surface de contact entre ladite couche de libération et la couche adhésive a été traitée avec un agent de libération.

10. Feuille adhésive selon la revendication 9, dans laquelle ledit agent de décollage est un matériau de silicone.

11. Feuille adhésive selon la revendication 7 utilisée pour l'application à une partie nasale.

12. Feuille adhésive nasale selon la revendication 11, dans laquelle au moins une paire de marques de positionnement est disposée à l'extrémité supérieure et/ou à l'extrémité inférieure de ladite feuille adhésive symétriquement par rapport à la ligne centrale de la feuille adhésive.

13. Feuille adhésive nasale selon la revendication 11, dans laquelle au moins une paire de marques de positionnement est disposée à la portion de l'extrémité supérieure et/ou la portion de l'extrémité inférieure de la feuille adhésive symétriquement par rapport à la ligne centrale de la feuille adhésive.

14. Procédé d'utilisation d'une feuille adhésive selon la revendication 2, comprenant l'étape consistant à amener la surface de la couche adhésive de la feuille adhésive à un état humidifié ou humide avant l'application en l'amenant en contact avec de l'eau ou un milieu hydrophile.

15. Procédé d'utilisation d'une feuille adhésive selon la revendication 2, qui comprend l'humidification d'un site d'application avec de l'eau ou un milieu hydrophile à l'avance et l'application de la surface de la couche adhésive de la feuille adhésive sur le site d'application.

*FIG. 1*

21    21

A

11

*FIG. 2*

22    22

A

11

*FIG. 3*

23    23

A

11

FIG. 4

FIG. 5

FIG. 6

*FIG. 7*

23a    23a

A    11

23b    23b

*FIG. 8*

31a    31a

B    11

31b    31b

*FIG. 11*

C

FIG. 9 (a)

32    32

B

11

FIG. 9 (b)

32    32

B

11

12

FIG. 10

33    33

B

11

12